# EUROPEAN PATENT APPLICATION

(11) **EP 1 878 407 A1**
(43) Date of publication of application: **16.01.2008**
(21) Application number: 07386016.5
(22) Date of filing: 09.07.2007
(51) Int. Cl.: A61F 2/24, A61L 27/00

(54) **Human cardiac valve replacement with seal heart valve (aortic or pulmonary)**

(30) Priority: 12.07.2006 GR 20060100400
(71) Applicant: Agathos, Efstathios-Andreas, 152 37 Filothei-Attikis (GR)
(72) Inventor: Agathos, Efstathios-Andreas, 152 37 Filothei-Attikis (GR)

(57) **Abstract**

The present invention provides a new method of constructing a tricuspid bioprosthetic valve derived from the heart valve of seal (aortic or pulmonary), which can be used to treat human cardiac valve dysfunction, by replacing the exixting valve with a novel bioprosthesis of the present invention. This new bioprosthesis, after having being extracted from the heart of the seal, is treated with a fixative agent to sterilize, fix, increase the strength of the leaflets and lessen the antigenicity of the tissue valve. Following this step the seal valve can either be stent-mounted or can be unstented. Beeing a natural trileaflet valve, with symmetrical cusps and no muscle shelf within the leaflet, the seal valve presents superior hemodynamic performance over the existing aortic porcine and bovine pericardial valves. The increase thickness of the leaflets result in a long term durability, without the attendant disadvantages or re-replacing a bioprosthesis. Alternatively, a seal valve can be constructed from tissues such as pericardium or fascia lata.

## Description

### FIELD OF INVENTION

This invention relates to the field of surgical implants, and in particular to a method of treating cardiac valve dysfunction in a human patient by replacing the existing valve with a seal valve (aortic or pulmonary).

### BACKGROUND

The human heart valve is a four chambers muscular organ, which serves as the main pump of blood in the circulatory system. Systemic venous blood enters the the right atrium through the superior and inferior vena cavae: then through the tricuspid valve enters the right ventricle where it is pumped to the pulmonary artery through the pulmonary valve. Blood from the lungs enters the left atrium through the four pulmonary veins: then through the mitral valve enters the left ventricle and through the aortic valve into the aorta and the rest of the body.

The function of these valves is to allow the blood flow easily through them in one direction by opening the leaflets and preventing blood from returning back by closing the leaflets. In some individuals one or more valves may not function normally, usually as a result of disease-induced valve damage, degeneration or a congenital defect, with the attendant result of stenosis or regurgitation. Both conditions can lead to life threatening situation. Over the last 47 years , severe valve dysfunction has been treated by replacing the diseased valve with a mechanical prosthesis, or alternatively, with a tissue valve (i.e. valve of human or animal tissue). Tissue valves have the advantage of low thrombogenicity, therefore they do not need anticoagulation, as this is the case for mechanical valves, thus eliminating the episodes of hemorrhage and thromboembolic events and lower incidence of serious infection of the heart, such is infective endocarditis. Tissue valves can be categorized as allografts (usually aortic valves from cadavers, also referred as homografts) or xenografts (animal heart valves). In addition some some aortic valves have been replaced by autograft (Ross procedure), which is a pulmonary valve from the same patient which in turn is replaced with an allograft (homograft) or a xenograft ("Tissue Heart Valves", ed. by M. I. Ionescu, publisher Butterwoth Inc., Boston Mass., U.S.A., 1979, particularly at pp. 146-172). Due to difficulties in sterilization, storage and availability of multiple valve sizes, allografts (homografts) are not widely used by cardiac surgeons. Ross procedure is a lengthy and technically very demanding operation not widely used either. Xenografts from the other hand are quite commonly used by cardiac surgeons. These are either porcine aortic valves or valves constructed by bovine pericardium (R.E. Jamieson: J. Card. Surg., 1993;8;89-98). A variety of sizes are readily available. Both types of xenografts have to be first treated with an agent, typically glutaraldehyde, to fix the valve tissue, sterilize it, and decrease its antigenicity. In particular the porcine aortic xenograft has been used both as stented (mounted in a frame) or unstented ("Tissue Heart Valves", supra, particularly at pp. 32-34, 107-109, and 177). Stentless valves increase the effective orifice of the valves but their surgical implantation is more technically demanding and can be used only at the aortic position. The porcine aortic valve is tricuspid, but there is an obvious disadvantage, which is the presence of a muscle shelf at the non-coronary cusp, thus preventing the valve in fully opening and so reducing the hemodynamic performance of the valve. Pericardial bovine valves are not preferred at the mitral position, due to the increase risk of thromboembolic events and accelerated structural failure (C.G. Duran, in "Replacement Cardiac Valves" edited by E. Bodnar & R Frater, publisher Pergamon Press, Inc., New York 10523 U.S.A., 1991, pp. 277-285). Both porcine aortic and bovine pericardial valves start to fail five years after surgical implantation and eventually all require re-replacement in approximately 10 years (G. I. Grunkemeier et al., Curr. Probl. Cardiol. 1992:17:362-368). The use of porcine pulmonary valve as was suggested by Ross (U.S. Pat. No. 5,352,240, issued in 1994) has not been widely used by cardiac surgeons, as the pulmonary leaflets are very thin and friable and soon fail after the surgical implantation. The Wain bioprosthetic valve (United Kingdom Patent Application No. 8303479, Publication No. 2 136533A, filled by Wain in 1983) represents a valve too cumbersome to manufacture, without any clear advantage over the existing bioprosthetic valves and without proven durability. New animals have been tested such as Kangaroo (C. Weinhold et al., Z. Kardiol. 75, Suppl. 2, pp. 251-253, 1986), and although promising the limited animal stock has been failed to cover the international market. In addition to the aforementioned limitations, porcine aortic or pulmonary valves are not favored by Muslim patients, while bovine pericardial valves are not favored by Buddhists patients and other similar religious groups, thereby excluding a large portion of the world market.

### BASIC PRINCIPLES OF THE INVENTION

The present invention provides a method of treating diseased human cardiac valves, by surgically replacing the existing valve with a novel bioprosthesis of the present Invention, which comprises a seal valve (aortic or pulmonary). Preferably, the seal valve is treated with a fixative, such as glutaldehyde, polyepoxy compound or any other proper agent to fix, sterilize, increase the strength of the leaflets and reduce the antigenicity of the valve tissue. Following this step the seal valve can either be stent-mounted or can be unstented. The seal valve has superior hemodynamic performance than the existing porcine aortic or bovine pericardial valve and in addition better durability due to the increase thickness of the leaflets. Due to the fact that the seal leaflets contain more collagen and elastic fibers than the existing bioprosthetic valves, the seal valve becomes more durable, thus eliminating the risk of reoperation, which carries a high risk of complications and mortality. Additional objects and advantages of this novel bioprosthesis is the acceptance from Muslims (Arabic countries) and Buddhists (i.e. India), which represent a very large part of the world market. As the seal valves can be harvested from cadaveric animals (i.e. those accidentally caught during commercial fishing activities, or during population control programs in Canada), the source of this bioprosthetic valve becomes inexpensive, maintaining an ample supply (an estimated 450,000 valves per year), large enough to supply the international market). Moreover breeding programs can be started in countries with the suitable climatological conditions, such as Canada, Russia, Argentina and others.

### BRIEF DESCRIPTION OF THE DRAWINGS

FIG. **1** is a perspective view of an unstented seal aortic valve with an attached segment of ascending aorta, showing the presence of a sewing skirt which has has been sutured adjacent the inflow side of the valve.
FIG. **2** is a perpective view of an unstented seal pulmonary valve with an attached segment of pulmonary artery, showing the presence of a sewing skirt which has been sutured adjacent the inflow side of the valve.
FIG. **3** is a perspective view of a stent-mounted seal valve (aortic or pulmonary).
FIG. **4** is a perspective view of a stent-mounted seal valve (aortic or pulmonary) from the inflow side.

### REFERENCE NUMERALS

**6** seal aortic valve
**8** aortic annulus
**10** cusps of leaflets
**12** segment of the seal ascending aorta
**14** orifice of the right coronary artery
**16** orifice of the left coronary artery
**18** flexible sewing ring
**20** seal pulmonary valve
**22** pulmonary annulus
**24** segment of pulmonary artery
**26** conventional stent
**28** flexible legs
**30** sewing ring

### DESCRIPTION OF THE INVENTION AND EMBODIMENTS

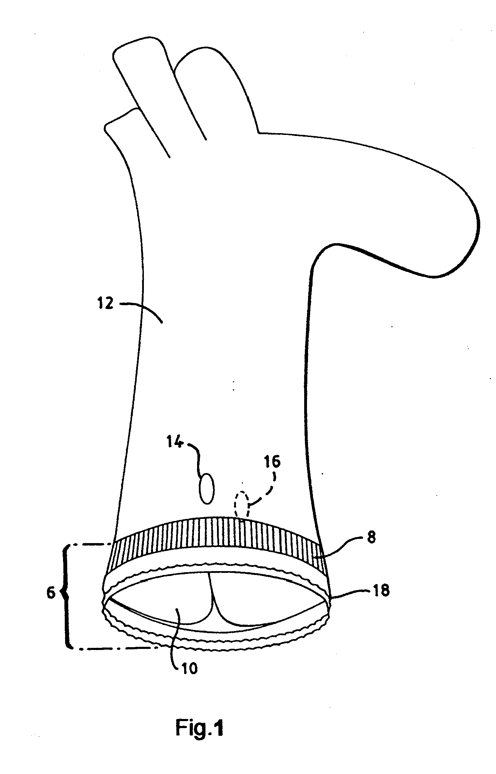

FIG. **1** shows an unstented seal aortic valve. An excised seal aortic valve **6** is shown, which actually consists of an aortic annulus **8** and three cusps or leaflets **10** attached to the annulus **8.** A segment of the seal ascending aorta **12** has been excised along with the aortic valve **6.** The two coronaries arteries have been excised and the orifice of the right coronary artery **14** and the orifice of the left coronary artery **16** have been slightly enlarged. At this point the unitary combination of the valve **6** along with the segment **12** would be treated with a fixative such as glutaraldehyde, a polyepoxy compound, or any other suitable agent, to fix and decrease the antigenicity of the tissue. An unstented bioprosthesis can then be prepared by suturing a flexible sewing ring **18,** made of synthetic polymer, such as those sold under the trademarks DACRON or TEFLON, to the aortic annulus **6.** This unstented seal aortic bioprosthesis can then be sterilized and stored in the same manner as known for unstented porcine aortic valves.

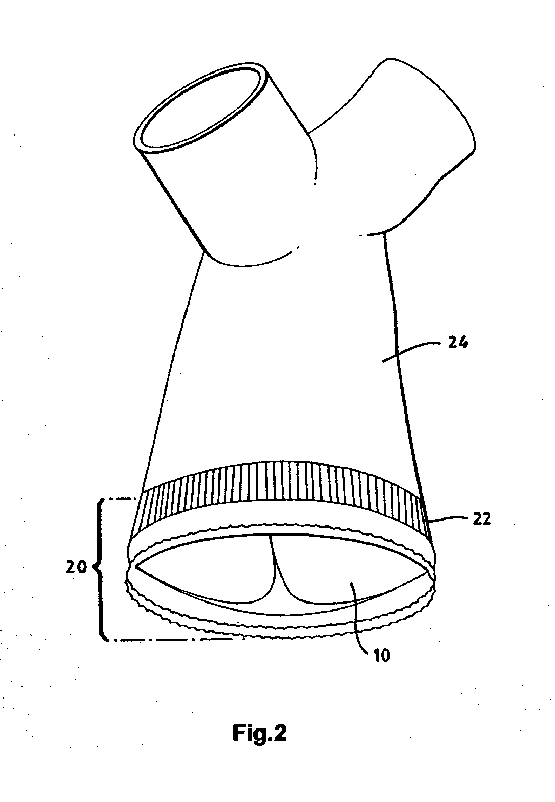

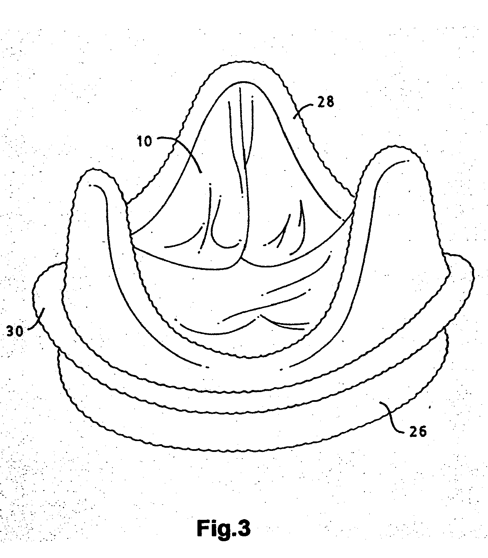

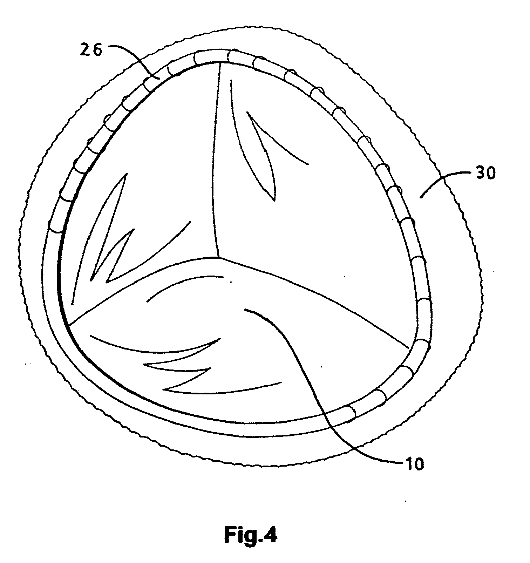

FIG. **2** shows an unstented seal pulmonary bioprosthesis. An excised seal pulmonary valve **20,** is shown, which actually consists of a pulmonary annulus **22** with three cusps or leaflets **10** attached to it.A segment of pulmonary artery **24** has been excised along with the pulmonary valve **20.** At this point the unitary combination of the valve **20** along with the segment **22** can be treated in exact the same way as was described before for the unstented seal aortic bioprosthesis. This unstented seal pulmonary bioprosthesis can then be sterilized and stored in the same manner as known for unstented porcine aortic valves. A stented seal bioprosthesis (essentially same design characteristics for both the aortic and the pulmonary valve) is shown in FIG. **3** and FIG. **4** (inflow view). After having fixed the tissues, as has been described above, the segment of the ascending aorta **12,** or the segment of the pulmonary artery **24** is removed, and the remaining valve structure **6** or **20** is sutured to a conventional stent **26,** which is made of metal or any other suitable material. This stent **26** has three upstanding, symmetrical and inwardly flexible legs **28** and a conventional sewing ring **30** to facilitate implantation. Both the structure of stent **26** and the manner of suturing the seal valve (aortic **6** or pulmonary **20**) thereto, are analogous to the well known stents and suturing techniques used to prepare a stented porcine aortic valve. For example, see "Tissue Heart Valves", supra., particularly at pp. 32-34, 107-109 and 177. The resulting stented seal valve can then be sterilized and stored in the same manner, and under the same conditions, as the unstented valves of FIG. **1** and **FIG. 2.** The resulting fixed and sterile seal valves, both stented (FIG. **3** and FIG. **4),** and unstented with a sewing skirt (FIG. **1** and FIG. **2),** can then be used to replace human cardiac valves with well known and established surgical techniques. For example, the unstented seal valve with sewing skirt (FIG. **1** and FIG. **2)** can replace a human aortic valve using essentially the same techniques as for replacement of a human aortic valve with an allograft or homograft. For example, see "Tissue Heart Valves", supra., particularly at pp. 137-150, and "The Aortic Valve", ed. Emery and Arom, publisher Hanley & Belfus Inc., Philadelphia, PA, U.S.A. (1991), in particular pp. 219-225. In the same manner, the stented seal valve (FIG. 3 and FIG. **4)** can be used to replace a human aortic valve using essentially the same surgical technique as used to replace a human aortic valve with a stented porcine aortic valve. See, for example, "Tissue Heart Valves", supra., particularly at p. 122, and "Cardiac Surgery", eds. John W. Kirklin & Brian G. Barratt-Boyes, published by Churchill Livingstone Inc., New York, N.Y., U.S.A., 2nd edition (1993), and particular at p. 511. The stented seal valve (aortic or pulmonary) is mainly recommended for replacement of human aortic, mitral and tricuspid valves. The unstented seal valve (aortic or pulmonary) is mainly recommended for replacement of human aortic or pulmonary valves.

### PREFERRED EMBODIMENT-OPERATION

The seal valves (aortic and pulmonary) are naturally trileaflet valves. The leaflets have a thick layer of collagen and elastic fibers, they lack of muscle shelf (i.e., as is the case in the porcine aortic valves) thus presenting no obstruction to bloodflow opening in full extent with the minimum of work required, and having an excellent coaptation in the close position, preventing therefore any backflow (leakage). For example, the aortic valve of an adult harbor seal (Phoca Vitulina) was tested for hemodynamic performance in the laboratory. This heart was donated to me by the Northeast Fisheries Science Center, in Woods Hole, Mass., U.S.A. under the Marine Mammal Protection Act Permit #979. This animal was an incidental catch during commercial fishing operations in the Gulf of Main, U.S.A., having been maintained in the frozen state of -20 degrees of Celcius. The aortic valve was then harvested as a fresh xenograft and mounted to a silicone rubber tube. The unitary combination of the aortic valve and silicone tube was then placed in a "pulse duplicator" which is a standard valve tester complying to the requirements of the International Standards Organization (ISO): ISO 5840, Cardiovascular Implants-Cardiac Valve Prosthesis. The test followed the Federal Drug Administration (FDA) guidelines for new valve tests, which is to simulate hemodynamic conditions similar to that of an adult human normal functioning heart under different level of activity. Therefore the simulation is that of a beatig heart at 70 beats per minute, with an aortic pressure of 100 mmHg, a ventricular pressure of 50 mmHg and a cardiac output (volume of blood pumped by minute) of 3, 4 and 5 liters per minute. For each cardiac output there were 10 different measurements and the computerized results were as shown below:

| Cardiac Output (L/min) | 3 | 4 | 5 |
|---|---|---|---|
| Transaortic Pressure Gradient (mmHg) | 0.93 | 0.24 | 1.93 |
| Leakage Backflow (%) | 3.42 | 2.93 | 2.60 |
| Leakage Volume (ml) | 1.81 | 2.02 | 2.17 |
| Closing Energy (JouiesxlE-3) | 52.70 | 65.30 | 71.90 |
| Effective Aortic Orifice (cm²) | 3.46 | 3.76 | 3.76 |

These results show the excellent hemodynamic performance of the seal valve which is superior to any other bioprosthetic valve tested before. An other obvious advantage of the present invention is the thicker consistency of the leaflets (due to the higher level of collagen and elastic fibers) making them more durable than the existing bioprosthetic valves. It is of importance to stress that the cadaveric seal valves (if harvested within 20 hours of the animal death), retain all of the superb functions and characteristics and as so there is no need for sacrificing live animals for this purpose. Furthermore the use of seal valves as a natural byproduct, will eliminate the commercial cost of these novel bioprosthesis making it more compatible with the existing bioprostheses. The large amount of incidentally killed animals during commercial fishing in the U.S.A. (about 40,000 per year), or i.e. the annual seal harvesting supported by Canadian Government for "population control" (about 450,000 per year) guarantee the stock of supply of this new bioprosthetic valve also.
As all seals share essentially identical cardiac valve anatomy, the following species are included under the term "seal" of the present invention: Harbor Seal (Phoca Vitulina), South American Fur Seal (Arctocephalus Australis), New Zealand Fur Seal, Western Australian Fur Seal (Arctocephalus Fosteri), Galapagos Fur Seal (Arctocephalus Galapogoensis), Juan Fernadez Fur Seal (Arctocephalus Philippii), Kerguelen Fur Seal, Antarctic Fur Seal (Arctocephalus Gazella), South African Fur Seal, Cape Fur Seal (Arctocephalus Pusillus), Amsterdam Island Fur Seal, Subantarctic Fur Seal (Arctocephalus Tropicalis), Northern Fur Seal (Callorhinus Ursinus), Hooded Seal, Bladdernose Seal (Cystophora Cristata), Bearded Seal (Erignathus Barbatus), California Sea Lion (Zalophus Californianus), Northern Sea Lion, Stella Sea Lion (Eunetopias Jubatus), Gray Seal (Halichoerus Grypus), Leopard Seal (Hydrurga Leptonyx).
It will be appreciated that modifications to the embodiments described in detail above, are of course possible. Accordingly, the present invention is not limited to the embodiments which have been described in detail above. For example, seal tissues such as pericardium or fascia lata can be used to construct trileaflet bioprostheses, with techniques for example similar to those used for construction of bovine pericardial valves. Accordingly, it can be seen that the present invention provides a method of treating human cardiac valve dysfunction, by replacing the existing valve with a novel bioprosthesis of the present invention, which comprises a seal valve (aortic and pulmonary). Preferably the seal valve has been treated with a fixative agent, such as glutaradehyde, polyepoxy compounds or any other suitable agent to fix the tissue valve, sterilize and at the same time to increase the valve strength and reduce the antigenicity of the tissue. The seal valve can be either stent-mounted by techniques analogous to that by which the already existing porcine aortic or bovine pericardial valves are stented, or it can be unstented.
The present invention therefore provides a bioprosthesis, comprising a seal valve (aortic and pulmonary), which when unstented, is suitable for replacing the human aortic or pulmonary valves, or which, when stented, is suitable for replacing the human aortic, mitral or tricuspid valves. The method of replacing the human valves with the seal valve offers all the advantages of using bioprosthetic versus mechanical valves; in addition, eliminates a major disadvantage of the existing porine aortic valves, which is the asymmetry of the cusps and the presence of a muscle shelf therein which results in restricted blood flow, and the disadvantage of the bovine pericardial valves that being artificial cannot match the function and the properties of natural valves. Due to the thickness of the cusps or leaflets, the seal valve (aortic and pulmonary) will result in a valve of sufficiently increased durability for replacing the human valve, without the attendant disadvantages of a porcine aortic or bovine pericardial valve replacement.

## Claims

1. A method of constructing a new bioprosthetic cardiac valve, which concists of trileaflet seal valve.

2. The method of claim 1, wherein said seal valve is a fixed and sterilized stentless trileaflet valve.

3. The method of claim 1**,** wherein said seal valve is a fixed and sterilized stent-mounted trileaflet valve.

4. The method of claim 1, wherein said seal valve is constructed from seal tissues, such as pericardium or fascia lata.

5. The method of claim 1, wherein said seal valve comprises a section of ascending aorta.

6. The method of claim 1, wherein said seal valve comprises a section of pulmonary artery.
